# EUROPEAN PATENT APPLICATION

(11) **EP 1 905 462 A1**
(43) Date of publication of application: **02.04.2008**
(21) Application number: 07253819.2
(22) Date of filing: 26.09.2007
(51) Int. Cl.: A61L 27/38

(54) **Hollow tissue growth devices made by lyophilization**

(30) Priority: 29.09.2006 US 847921 P
(71) Applicant: Johnson & Johnson Regenerative Therapeutics, LLC, Raynham, MA 02767 (US)
(72) Inventor: Keeley, Daniel, Boston, MA 02215 (US); Shetty, Dhanuraj, Somerset, NJ 08873 (US); Hammer, Joseph J., Hillsborough, NJ 08844 (US); Wang, Ziwei, Monroe Twp, NJ 08831 (US)
(74) Representative: Belcher, Simon James

(57) **Abstract**

A device for organ replacement and regenerative medicine comprises a biocompatible and biodegradable scaffold which is capable of integral cell growth and which is in the form of a hollow chamber. Such devices can be produced by lyophilizing biocompatible, biodegradable polymers to produce a seamless, three-dimensional shape.

## Description

The present invention relates to devices and methods for organ replacement and regenerative medicine. More specifically, the present invention provides for a hollow chamber formed by a biocompatible and biodegradable scaffold capable of integral cell growth that may facilitate the regeneration of an organ.

Regenerative medicine is a developing field targeted at treating disease and restoring human tissues. Potential therapies may prompt the body to autonomously regenerate damaged tissue. Additionally, tissue engineered implants may also prompt regeneration. Developing approaches may also enable direct transplantation of healthy tissues into a damaged-tissue environment.

Many of these new therapies may require implantable biocompatible and biodegradable scaffolds for use both in vitro and in vivo. These scaffolds may augment healing through tissue infiltration or by providing suitable means of cell attachment and proliferation. Hollow chambers comprising biocompatible and biodegradable scaffolds are unique in that they may have the ability not only to replace damaged tissue but to replace entire organs. During 2001, at least 80,000 persons awaited organ transplants, but less than 13,000 transplants were made available. Hence, there remains a huge unmet need for appropriate biocompatible and biodegradable scaffolds upon which entire human organs or tissues can grow or regenerate.

Biocompatible scaffold fabrication methods are challenged in their ability to produce effective scaffolds from a limited number of materials. At the moment, one of the greatest challenges lies in producing a mechanically stable scaffold with high enough porosity to augment healing through cell proliferation and tissue ingrowth. There is also a lack of adequate methodologies to make these scaffolds into hollow structures. These and other deficiencies in the prior art are overcome by the present invention.

The present invention provides a tissue growth device for organ replacement and regenerative medicine comprising a biocompatible, biodegradable scaffold capable of integral cell growth that forms a hollow chamber.

Preferably, the biocompatible, biodegradable scaffold that forms the hollow chamber is produced by lyophilizing a biocompatible, biodegradable polymer to create a seamless, three-dimensional shape. In this regard, the lyophilizing methodology may include placing a solution comprising a biocompatible, biodegradable polymer dissolved in sublimable solvent into a mould; applying force to distribute said solution substantially evenly within a mould; freezing said solution within said mould wherein the resulting frozen construct has an exterior shape and texture consistent with the mould's internal geometry; and removing solvent via vacuum sublimation.

The tissue growth device of the invention can include biological factors, such as growth factors, hormones and cytokines, or drugs, such as antibiotics, analgesics and anti-inflammatory agents, or combinations thereof.

The device of the invention can be used to provide tissue which is generated by a growth device for organ replacement and regenerative medicine.

Embodiments of the invention are described below by way of example with reference to the accompanying drawings, in which:

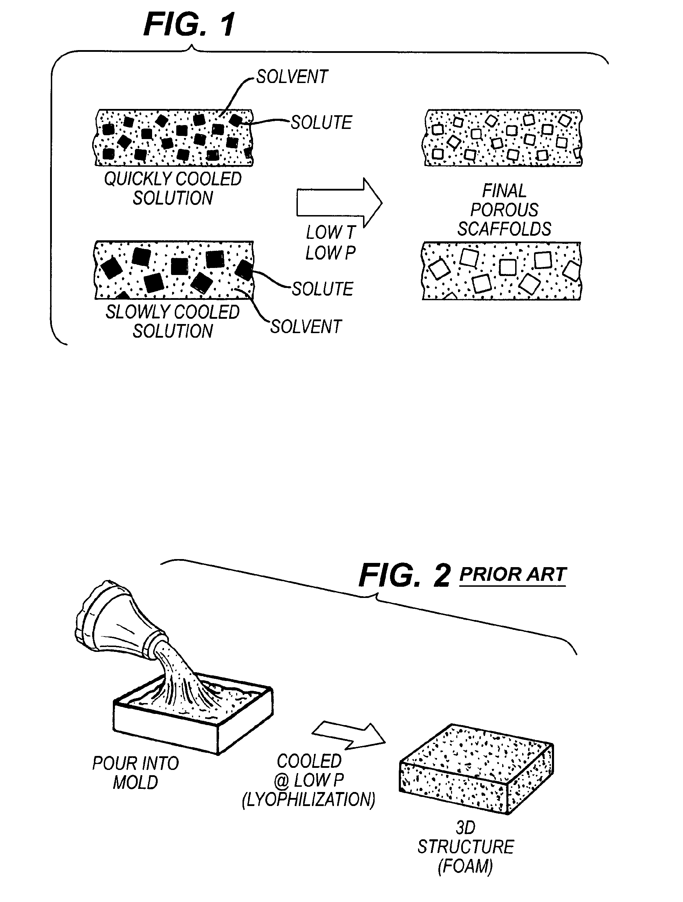

Figure 1 depicts an embodiment of the present invention in which solvent of a polymer-solvent solution is separated out via lyophilization, leaving a porous, polymer scaffold made out of the remaining solute.
Figure 2 depicts a limitation of existing technology whereby liquid solutions are poured into moulds before freezing and can only create solid and not hollow constructs of various shapes.
Figures 3, 4, and 5 depict an embodiment of the present invention in which liquid solutions are poured into moulds before freezing and mechanically rotated during freezing to produce a three-dimensional, seamless hollow structure.

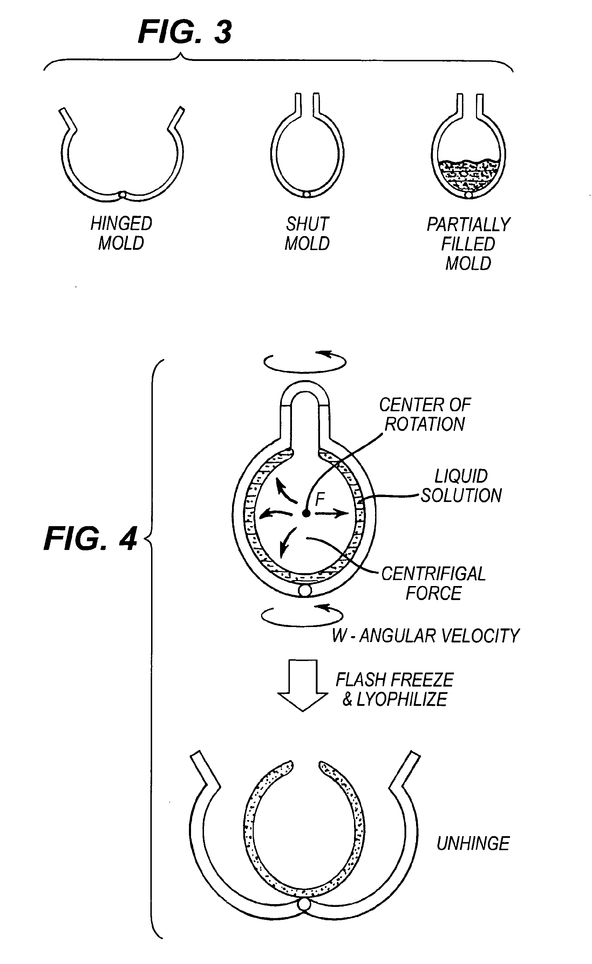

Figure 3 depicts the first step in an embodiment of the present invention in which the mould is hinged shut and partially filled with solution.
Figure 4 depicts an embodiment of the present invention in which the partially-filled mould is held vertically and spun quickly whereby centrifugal force acts on the liquid solution and pushes the solution away from the centre of the mould and up on its sides.

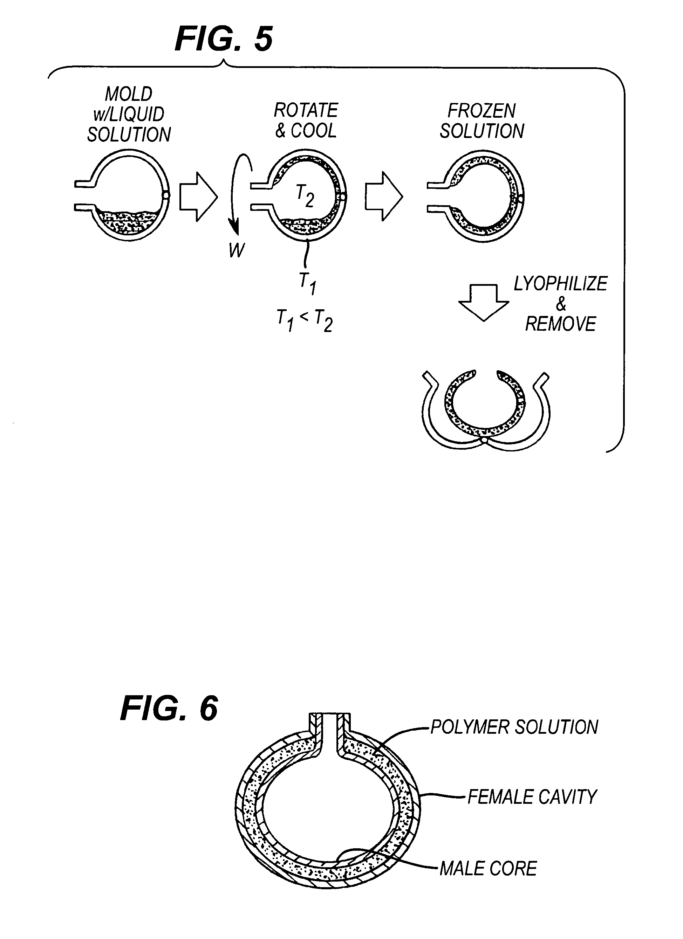

Figure 5 depicts an embodiment of the present invention in which the partially-filled mould is held horizontally and rotated slowly whereby gravity allows the polymer to settle upon one side of the mould.
Figure 6 depicts an embodiment of the present invention in which a hollow construct is made by a two-step mould where one part of the mould consists of a hollow section and the other part, a core.

Other than in the operating examples, or where otherwise indicated, all numbers expressing quantities of ingredients or reaction conditions used herein should be understood as modified in all instances by the term "about." The term "about" when used in connection with percentages may mean ± 1%.

The present invention provides for a device for organ replacement and regenerative medicine comprising a biocompatible, biodegradable scaffold capable of integral cell growth that forms a hollow chamber. The scaffold may also act as a substrate or carrier for cells, growth factors, bioactives, and drugs.

Regarding the "hollow chamber," the device may consist of a single chamber that is hollow or substantially hollow. Alternatively, the device may consist of more than one chamber that is hollow or substantially hollow. The chambers may or may not be attached to each other. Indeed, the invention contemplates an aggregate of individual hollow chambers as well as a subdivided single chamber. "Integral cell growth" refers to the process including, but not limited to, cell attachment, proliferation, differentiation, infiltration, residence, and outgrowth such that as the scaffold degrades, tissue growth and organ regeneration may give rise to a biologically functioning tissue or organ.

Lyophilization, or freeze-drying, removes a solvent from a polymer-solvent solution through sublimation, leaving behind a porous solid. More specifically, the process separates a solvent from a frozen solution through a solid to gas phase transition. This transition, called sublimation, removes the solvent without it ever entering a liquid state. The final construct is a porous solid structure made out of the remaining solute often described as a foam.

Recent publications have discussed using lyophilization to produce foam scaffolds. US-6333029 and US-6306424 disclose methods for dissolving various polymers and manufacturing them into porous foams having "a gradient in composition and/or microstructure" through lyophilization. US-6905105, US-A-2003/0171705, and US-A-2005/0221484 disclose a device that "prepares a biocompatible biodegradable matrix capable of supporting cells to form an implantable or engraftable surgical device" by filling a chamber with "matrix-forming fluid" and then cooling the chamber at a controlled rate. These inventions are not based on a hollow structure.

Liquid solution comprising any natural or synthetic biocompatible, biodegradable polymer, or any blend of such polymers, dissolved in a solvent that can be removed through sublimation, is poured into an open-ended, hinged mould and mechanically rotated during freezing. This invention is applicable to any such solution. In the first step, the mould is hinged shut and partially filled with solution. See Figure 3. During filling, some of the mould's volume remains empty. After lyophilization, the volume of solution poured into the mould will make up the scaffold volume whereas the empty volume will make up the hollow void. After filling, the mould may be rotated in a number of ways. When the mould is held vertically and spun quickly, a centrifugal force acts on the liquid solution, pushing it away from the mould's centre and up upon its sides. The spinning mould may then be cooled slowly or flash frozen by submersion in liquid nitrogen. See Figure 4. The mould may also be held horizontally and rotated slowly whereby gravity allows the polymer to settle upon one side of the mould. Assuming that the temperature of the mould is lower than the temperature of the ambient air, a layer of frozen liquid will gradually build up on the mould's interior, resulting in an internal frozen skin. Both methods will produce a frozen construct that has a shape and texture consistent with the mould's internal geometry. Once fully frozen, the construct is placed in a vacuum for sublimation.

A variety of absorbable polymers can be used to make foams. Examples of suitable biocompatible, bioabsorbable polymers that could be used include polymers selected from the group consisting of aliphatic polyesters, poly(amino acids), copoly(ether-esters), polyalkylene oxalates, polyamides, poly(iminocarbonates), polyorthoesters, polyoxaesters, polyamidoesters, polyoxaesters containing amine groups, poly(anhydrides), polyphosphazenes, biomolecules (i.e., biopolymers such as collagen, elastin, bioabsorbable starches, etc.), and blends thereof.

Suitable solvents include but are not limited to solvents selected from a group consisting of formic acid, ethyl formate, acetic acid, hexafluoroisopropanol (HFIP), cyclic ethers (i.e., THF, DMF, and PDO), acetone, acetates of C₂ to C₅ alcohol (such as ethyl acetate and t-butylacetate), glyme (i.e., monoglyme, ethyl glyme, diglyme, ethyl diglyme, triglyme, butyl diglyme, and tetraglyme), methylethyl ketone, dipropyleneglycol methyl ether, lactones (such as γ-valerolactone, δ-valerolactone, β-butyrolactone, γ-butyrolactone), 1,4-dioxane, 1,3-dioxolane, 1,3-dioxolane-2-one (ethylene carbonate), dimethylcarbonate, benzene, toluene, benzyl alcohol, p-xylene, naphthalene, tetrahydrofuran, N-methyl pyrrolidone, dimethylformamide, chloroform, 1,2-dichloromethane, morpholine, dimethylsulfoxide, hexafluoroacetone sesquihydrate (HFAS), anisole and mixtures thereof. A homogenous solution of the polymer in the solvent is prepared using standard techniques.

As will be appreciated by those skilled in the art, the applicable polymer concentration or amount of solvent which may be utilized will vary with each system. Suitable phase diagram curves for several systems have already been developed. However, if an appropriate curve is not available, this can be readily developed by known techniques. The amount of polymer will depend to a large extent on the solubility of the polymer in a given solvent and the final properties of the foam desired.

A parameter that may be used to control foam structure is the rate of freezing of the polymer-solvent solution. The type of pore morphology that gets locked in during the freezing step is a function of the solution thermodynamics, freezing rate, temperature to which it is cooled, concentration of the solution, homogeneous or heterogeneous nucleation, etc. Detailed description of such phase separation phenomenon can be found in the references provided herein. See A. T. Young, "Microcellular foams via phase separation," J. Vac. Sci. Technol. A 4(3), May/June 1986; S. Matsuda, "Thermodynamics of Formation of Porous Polymeric Membrane from Solutions," Polymer J. Vol. 23, No. 5, pp 435-444, 1991).

In another embodiment of the present invention, a hollow chamber is constructed by a two-step mould where one part of the mould consists of a hollow section and another part consists of a core. See Figure 6. This design is similar to that used in a typical injection moulding process. The solution can be filled via the space between the cavity and the core. The space can be determined by the thickness of the final construct. Once the filling is complete, the solution can be frozen by the steps above.

In various embodiments of the present invention, the polymers or polymer blends that are used to form the biocompatible, biodegradable scaffold may contain pharmaceutical compositions. The previously described polymer may be mixed with one or more pharmaceutical pharmaceuticals prior to forming the scaffold. Alternatively, such pharmaceutical compositions may coat the scaffold after it is formed. The variety of pharmaceuticals that can be used in conjunction with the scaffolds of the present invention includes any known in the art. In general, pharmaceuticals that may be administered via the compositions of the invention include, without limitation: anti-infectives such as antibiotics and antiviral agents; chemotherapeutic agents; anti-rejection agents; analgesics and analgesic combinations; anti-inflammatory agents; hormones such as steroids; growth factors; and other naturally derived or genetically engineered (recombinant) proteins, polysaccharides, glycoproteins, or lipoproteins.

Scaffolds containing these materials may be formulated by mixing one or more agents with the polymer used to make the scaffold or with the solvent or with the polymer-solvent mixture. Alternatively, an agent could be coated onto the scaffold, preferably with a pharmaceutically acceptable carrier. Any pharmaceutical carrier may be used that does not substantially degrade the scaffold. The pharmaceutical agents may be present as a liquid, a finely divided solid, or any other appropriate physical form. Typically, but optionally, they will include one or more additives, such as diluents, carriers, excipients, stabilizers or the like. In addition, various biologic compounds such as antibodies, cellular adhesion factors, and the like, may be used to contact and/or bind delivery agents of choice (such as pharmaceuticals or other biological factors) to the scaffold of the present invention.

The hollow chamber of the present invention may be useful in regenerating such organs as the bladder whereby the present invention is seeded or engrafted with cells, preferably those of the host. For example, primary rabbit urothelial cells (RUC) have been found to attach readily to unwoven polyglycolic acid polymers in vitro, survive, and grow in vivo (US-5851833). Some differentiated cell types, such as chondrocytes and hepatocytes, have been found to remain functionally differentiated and in some cases to expand in vivo on non-woven polyglycolic acid or polylactic acid polymers. The polymer fibres provide sites for cell attachment, the reticular nature of the polymer lattice allows for gas exchange to occur over considerably less than limiting distances, and the polymers evoke host cell responses, such as angiogenesis which promote cell growth.

Synthetic polymers can also be modified in vitro before use, and can carry growth factors and other physiologic agents such as peptide and steroid hormones, which promote proliferation and differentiation. The polyglycolic acid polymer undergoes biodegradation over a four month period; therefore as a cell delivery vehicle it permits the gross form of the tissue structure to be reconstituted in vitro before implantation with subsequent replacement of the polymer by an expanding population of engrafted cells.

To regenerate such organs as the bladder, the hollow chamber of the present invention may also be implanted without having cells seeded beforehand. The matrix may contain pharmaceuticals or proteins, e.g., antibodies attached to cell adhesion factors that promote cell attachment, proliferation, differentiation, infiltration, residence, and outgrowth such that once the scaffold degrades, a biologically functioning tissue or organ remains.

### EXAMPLES

### Example 1. Hollow Matrix Fabricated by Lyophilization

An open-ended, hinged mould is hinged shut and partially filled with a solution comprising a biocompatible, biodegradable polymer dissolved in sublimable solvent. The mould is then held vertically and spun quickly, whereby centrifugal force acts on the liquid solution and pushes it away from the mould's centre and up upon its sides. The spinning mould is subsequently flash frozen by submersion in liquid nitrogen. Once fully frozen, the construct is placed in a vacuum for sublimation.

### Example 2. Hollow Matrix Fabricated by Lyophilization

An open-ended, hinged mould is hinged shut and partially filled with a solution comprising a biocompatible, biodegradable polymer dissolved in sublimable solvent. The mould is then held horizontally and rotated slowly, whereby gravitational force allows the polymer to settle on one side of the mould. With the temperature of the mould lower than the temperature of the ambient air, a layer of frozen liquid gradually builds up on the mould's interior. This results in an internal frozen skin. Once fully frozen, the construct is placed in a vacuum for sublimation.

### Example 3. Hollow Matrix Formed by Two-Step Mould

A two-step mould is used to fabricate the hollow scaffold. The mould consists of a hollow section and a core. Solution is filled via the space between the cavity and the core. Once filling is complete, the solution is frozen by one of the steps described above.

## Claims

1. A tissue growth device comprising a biocompatible, biodegradable scaffold capable of integral cell growth that forms a hollow chamber.

2. The device of claim 1 wherein said scaffold is produced by lyophilizing a biocompatible, biodegradable polymer to create a seamless, three-dimensional shape.

3. The device of claim 1 wherein said scaffold is produced by the steps comprising:
a. placing a solution comprising a biocompatible, biodegradable polymer dissolved in sublimable solvent into a mould;
b. applying force to distribute said solution substantially evenly within a mould;
c. freezing said solution within said mould wherein the resulting frozen construct has an exterior shape and texture consistent with the mould's internal geometry; and
d. removing solvent via vacuum sublimation.

4. The device of claim 1 further comprising a pharmaceutical agent.

5. The device of claim 4 wherein the pharmaceutical agent is selected from the group consisting of antibiotics, antiviral agents, chemotherapeutic agents, anti-rejection agents, analgesics, anti-inflammatory agents, hormones, steroids, growth factors, proteins, polysaccharides, glycoproteins, and lipoproteins.

6. A tissue generated using the device of claim 1.

7. The tissue of claim 6 wherein said tissue is bladder tissue.
